# EUROPEAN PATENT APPLICATION

(11) **EP 3 379 475 A1**
(43) Date of publication of application: **26.09.2018**
(21) Application number: 18159037.3
(22) Date of filing: 28.02.2018
(51) Int. Cl.: G06Q 10/10

(54) **INFORMATION PRESENTATION APPARATUS AND INFORMATION PRESENTATION METHOD**

(30) Priority: 23.03.2017 JP 2017057129; 18.10.2017 JP 2017201620
(71) Applicant: Panasonic Intellectual Property Management Co., Ltd., Osaka-shi, Osaka 540-6207 (JP)
(72) Inventor: RAFII, Zarina, Osaka-shi, Osaka 540-6207 (JP); KUBOTANI, Hiroyuki, Osaka-shi, Osaka 540-6207 (JP)
(74) Representative: Grünecker Patent- und Rechtsanwälte PartG mbB

(57) **Abstract**

An information presentation apparatus includes an ingredient information acquirer that acquires a type and a first placement orientation of an ingredient placed on a counter, a recipe information acquirer that acquires a preparation method defined by a recipe, and a second placement orientation of the acquired type of the ingredient when the ingredient is prepared in accordance with the preparation method, a comparator that compares the second placement orientation with the first placement orientation, a generator that generates preparation guidance information indicating the preparation method and generates rotation guidance information that prompts a preparator to rotate the ingredient from the first placement orientation to the second placement orientation in accordance with comparison results provided by the comparator, and a presenter that presents the rotation guidance information and the preparation guidance information, generated by the generator.

## Description

### BACKGROUND

### 1. Technical Field

The present disclosure relates to an information presentation apparatus and an information presentation method.

### 2. Description of the Related Art

According to the technique disclosed in Japanese Unexamined Patent Application Publication No. 2010-191745, an ingredient placed on a counter is photographed to identify the type of the ingredient, and an image indicating a preparation method (such as a cutting method) that is determined from a recipe and applied to the ingredient placed on the counter is displayed in an overlay fashion on the ingredient.

### SUMMARY

In one general aspect, the techniques disclosed here feature an information presentation apparatus. The information presentation apparatus includes an ingredient information acquirer that acquires a type and a first placement orientation of an ingredient placed on a counter, a recipe information acquirer that acquires a preparation method, for the type of the ingredient, defined by a recipe, and a second placement orientation of the acquired type of the ingredient when the ingredient is prepared in accordance with the preparation method, a comparator that compares the second placement orientation acquired by the recipe information acquirer with the first placement orientation acquired by the ingredient information acquirer, a generator that generates preparation guidance information indicating the preparation method and generates rotation guidance information that prompts a preparator to rotate the ingredient from the first placement orientation to the second placement orientation in accordance with comparison results provided by the comparator that has compared the first placement orientation with the second placement orientation, and a presenter that presents the rotation guidance information and the preparation guidance information, generated by the generator.

The information presentation apparatus and the information presentation method of the disclosure may assist a preparator in a preparation operation.

It should be noted that general or specific embodiments may be implemented as a system, an apparatus, a method, a recording medium, or a computer program, or any selective combination thereof.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

### BRIEF DESCRIPTION OF THE DRAWINGS

Fig. 1 generally illustrates an information presentation apparatus of a first embodiment;
Fig. 2 is a block diagram illustrating an example of a configuration of the information presentation apparatus of the first embodiment;
Fig. 3 is a flowchart illustrating an example of a process performed by the information presentation apparatus of the first embodiment;
Fig. 4 illustrates an example of rotation guidance information;
Fig. 5 illustrates an example of a state in which preparation guidance information is displayed in an overlay fashion on an ingredient which is aligned with a second placement orientation;
Fig. 6 is a block diagram illustrating an example of a configuration of an information presentation apparatus of a second embodiment;
Fig. 7 is a flowchart illustrating an example of a process performed by the information presentation apparatus of the second embodiment;
Fig. 8 illustrates an example of movement guidance information;
Fig. 9 illustrates an example of a state in which preparation guidance information is displayed in an overlay fashion on an ingredient which is shifted to a second placement location and aligned with the second placement orientation;
Fig. 10 is a block diagram illustrating an example of a configuration of an information presentation apparatus of a third embodiment;
Fig. 11 is a flowchart illustrating an example of a process performed by the information presentation apparatus of the third embodiment;
Fig. 12 illustrates an example of holding location guidance information that is displayed in an overlay fashion on a holding location of the ingredient responsive to the degree of advance in the preparation of the ingredient;
Fig. 13 illustrates how a location where the holding location guidance information is displayed in an overlay fashion changes in response to the degree of advance in the preparation; and
Fig. 14 illustrates an example of a state in which an image indicating a preparation method is displayed in an overlay fashion on the ingredient which is aligned with an inappropriate direction.

### DETAILED DESCRIPTION

### Underlying Knowledge Forming Basis of the Present Disclosure

Inventors have found that related-art information presentation apparatuses have a problem described below.

If the orientation of an ingredient placed on a counter is aligned with a direction different from a placement orientation defined in a recipe in the information presentation apparatus disclosed in Japanese Unexamined Patent Application Publication No. 2010-191745, an image indicating a preparation method (preparation guidance information) is displayed in an overlay fashion on the ingredient that is aligned with the inappropriate direction.

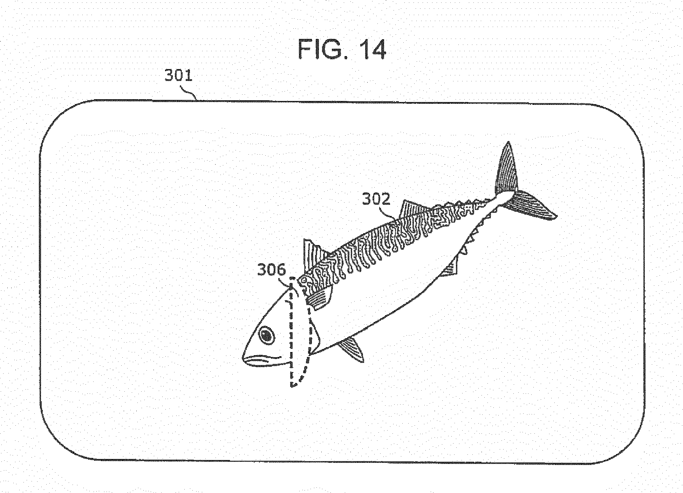

Fig. 14 illustrates an example of a state in which the preparation guidance information is displayed in an overlay fashion on an ingredient 302 that is aligned with an inappropriate placement orientation. Referring to Fig. 14, the ingredient 302 is mackerel placed on a cutting board 301. The preparation guidance information is an image 306 that indicates a cutting location where the mackerel is headed.

A beginner of preparing ingredients may possibly cut in a wrong way along the image 306 that indicates the cutting location that is displayed in an overlay fashion on the ingredient 302 that is aligned with an inappropriate placement orientation. By performing the wrong preparation method, the preparator may possibly get hurt.

The related-art information presentation apparatus does not account for the placement orientation of the ingredient that is placed and prepared on the counter, and is based on the premise that the ingredient is placed in the placement orientation defined in a recipe. For example, if the ingredient is not oriented as defined in the recipe, the image indicating the preparation method (preparation guidance information) is displayed in an overlay fashion on the ingredient that is inappropriately oriented, and the information presentation apparatus is unable to appropriately assist the preparator.

The related art techniques, such as the one disclosed in Japanese Unexamined Patent Application Publication No. 2010-191745, have not accounted for solutions in assisting the preparator safely and efficiently.

The disclosure provides an information presentation apparatus and an information presentation method for appropriately assisting the preparator in performing a preparation operation.

The information presentation apparatus of the disclosure includes an ingredient information acquirer that acquires a type and a first placement orientation of an ingredient placed on a counter, a recipe information acquirer that acquires a preparation method, for the type of the ingredient, defined by a recipe, and a second placement orientation of the acquired type of the ingredient when the ingredient is prepared in accordance with the preparation method, a comparator that compares the second placement orientation acquired by the recipe information acquirer with the first placement orientation acquired by the ingredient information acquirer, a generator that generates preparation guidance information indicating the preparation method and generates rotation guidance information that prompts a preparator to rotate the ingredient from the first placement orientation to the second placement orientation in accordance with comparison results provided by the comparator that has compared the first placement orientation with the second placement orientation, and a presenter that presents the rotation guidance information and the preparation guidance information, generated by the generator.

In this configuration, the rotation guidance information is presented to the preparator to prompt him or her to rotate the ingredient placed on the counter from the placement orientation (first placement orientation) to the placement orientation defined by the recipe (second placement orientation). The preparator may rotate the ingredient to the second placement orientation in accordance with the rotation guidance information. The ingredient placed on the counter is aligned with the second placement orientation, and the preparation guidance information is presented (for example, displayed in an overlay fashion) on the ingredient in the second placement orientation. The preparator is thus appropriately assisted in the preparation operation. This may possibly control an erroneous preparation operation of the preparator and help protect the preparator from getting hurt.

The information presentation apparatus may further include a placement orientation determiner that determines whether the first placement orientation and the second placement orientation, compared by the comparator, match each other. The presenter presents the rotation guidance information if the placement orientation determiner determines that the first placement orientation and the second placement orientation do not match each other. The presenter presents the preparation guidance information if the placement orientation determiner determines that the first placement orientation and the second placement orientation match each other.

In that configuration, the rotation guidance information is presented until the ingredient placed on the counter is changed from the first placement direction to the second placement orientation. The preparation guidance information is presented on the ingredient when the ingredient is rotated from the first placement orientation to the second placement orientation. Since the preparation guidance information is presented on the ingredient when it is determined that the ingredient is aligned with the second placement orientation, the information presentation apparatus may appropriately assist the preparator in the preparation operation.

The information presentation apparatus may further include a preparator information acquirer that acquires a standing location of the preparator. The ingredient information acquirer acquires a first placement location of the ingredient. The recipe information acquirer acquires a positional relationship between the standing location of the preparator when the acquired type of the ingredient is prepared in accordance with the preparation method defined by the recipe and a placement location of the acquired type of the ingredient. Based on the positional relationship acquired by the recipe information acquirer, the comparator calculates a second placement location concerning the standing location of the preparator that the preparator information acquirer has acquired, and compares the second placement location with the first placement location acquired by the ingredient information acquirer. The generator generates movement guidance information that prompts the preparator to move the ingredient from the first placement location to the second placement location, based on comparison results provided by the comparator that has compared the first placement location with the second placement location. The presenter presents the movement guidance information generated by the generator.

In that configuration, the movement guidance information is presented to the preparator to prompt him or her to move the ingredient from the placement location of the ingredient actually placed on the counter (the first placement location) to the placement location (the second placement location) corresponding to the positional relationship the standing location of the preparator defined by the recipe and the placement position of the ingredient. By moving the ingredient in response to the movement guidance information, the preparator may more easily prepare the ingredient.

The information presentation apparatus may further include a placement location determiner that determines whether the first placement location and the second placement location, compared by the comparator, match each other. The presenter presents the movement guidance information if the placement location determiner determines that the first placement location and the second placement location do not match each other. The presenter presents the preparation guidance information if the placement location determiner determines that the first placement location and the second placement location match each other.

In that configuration, the movement guidance information is presented until the ingredient has moved from the first placement location to the second placement location on the counter. The preparation guidance information is presented when the ingredient has been shifted from the first placement location to the second placement location. Since the preparation guidance information is presented on the ingredient when it is determined that the ingredient is placed at the second placement location, the information presentation apparatus may appropriately assist the preparator in the preparation operation.

The information presentation apparatus may further include a holding location calculator that calculates a holding location of the ingredient that accounts for a degree of advance of preparation of the ingredient when the ingredient is prepared in accordance with the preparation method. The ingredient information acquirer acquires the degree of advance of the preparation of the ingredient. The generator generates holding location guidance information that prompts the preparator to hold the ingredient at the holding location calculated by the holding location calculator. The presenter presents the holding location information generated by the generator.

In that configuration, the information presentation apparatus presents the holding location guidance information indicating the holding location of the ingredient that accounts for the degree of advance of the preparation in accordance with the preparation method defined in the recipe. The preparator may more easily prepare the ingredient, and may be protected from getting hurt.

The information presentation apparatus may further include a preparator information acquirer that acquires a standing location of the preparator. The ingredient information acquirer acquires a first placement location of the ingredient. The comparator calculates a presentation location where the rotation guidance information is presented, based on the first placement orientation acquired by the ingredient information acquirer, the first placement location, and the standing location of the preparator acquired by the preparator information acquirer. The presenter presents the rotation guidance information at the presentation location.

In that configuration, the information presentation apparatus may present the rotation guidance information at an appropriate location In accordance with the first placement orientation, the first placement location, and the standing location of the preparator. The preparator may more easily recognize the rotation guidance information.

The disclosure provides an information presentation method. The information presentation method includes acquiring a type and a first placement orientation of an ingredient placed on a counter, acquiring a preparation method, for the type of the ingredient, defined by a recipe, and a second placement orientation of the acquired type of the ingredient when the ingredient is prepared in accordance with the preparation method, comparing the acquired second placement orientation with the acquired first placement orientation, generating preparation guidance information indicating the preparation method and generating rotation guidance information that prompts a preparator to rotate the ingredient from the first placement orientation to the second placement orientation in accordance with comparison results of the first placement orientation with the second placement orientation, and presenting the generated rotation guidance information and the generated preparation guidance information.

In accordance with the disclosure, the preparation assisting method is provided to assist the preparator in a preparation operation.

The embodiments described below are general or specific embodiments of the disclosure. Numerical values, shapes, elements, steps and the order of the steps in the embodiments are described for exemplary purposes only, and are not intended to limit the disclosure. Among the elements in the embodiments, elements not described in the independent claims indicative of higher concepts may be described as optional elements.

The embodiments of the disclosure are described below with reference to the drawings.

### First Embodiment

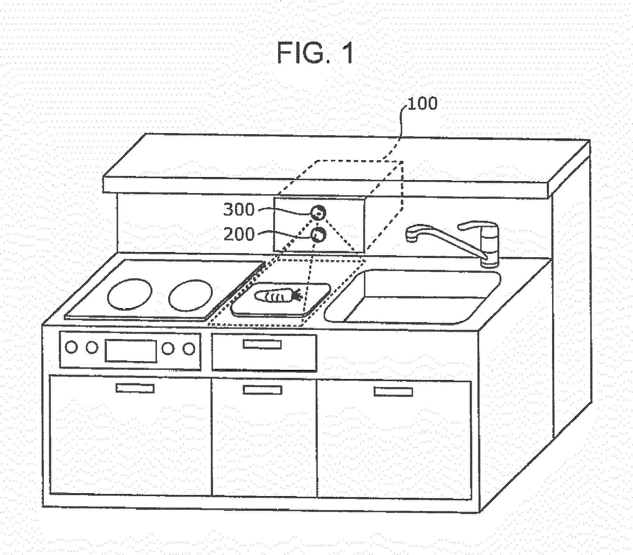

A first embodiment of the disclosure is described with reference to Fig. 1 through Fig. 5.

### 1.1 Configuration of information presentation apparatus

Fig. 1 illustrates an information presentation apparatus 100 of the first embodiment.

Referring to Fig. 1, the information presentation apparatus 100 may be installed in a system kitchen. The information presentation apparatus 100 may teach a preparator a preparation method, such as cutting an ingredient placed on a counter (such as a cutting board of Fig. 1). In accordance with the first embodiment, a preparation method is related to how to cut an ingredient placed on the counter. But the preparation method is not limited to this. The preparation method includes preparing the ingredient by processing it prior to baking, boiling, or frying the ingredient. Preparing the ingredient by processing it may include cutting the ingredient, spreading a seasoning on the ingredient, or flouring the ingredient. The preparator may be the same person as a cook. The information presentation apparatus 100 is installed on a deeper location than the counter if viewed from the standing location of the preparator (if viewed from the front side of the page of Fig. 1). The information presentation apparatus 100 may be installed at a cooking hood that overlooks the whole kitchen. The information presentation apparatus 100 may not be built in a system kitchen or a cooking hood but may be a separate unit that is installed later.

The information presentation apparatus 100 includes a camera 200 and a projector 300.

The camera 200 photographs ingredients and a preparator at or around the counter. The projector 300 projects an image to assist the preparator in the preparation operation at and around the counter. Fig. 1 illustrates a projection plane of the projector 300 at and around the counter. Since an image that is generated based on data that is obtained by the camera 200 photographing the counter and the surroundings thereof is projected from the projector 300, the preparator is assisted in the preparation operation.

The information presentation apparatus 100 may not necessarily include the camera 200 and the projector 300. The camera 200 and the projector 300 may be modules separate from the information presentation apparatus 100. For example, the camera 200 may be mounted on glasses worn by the preparator.

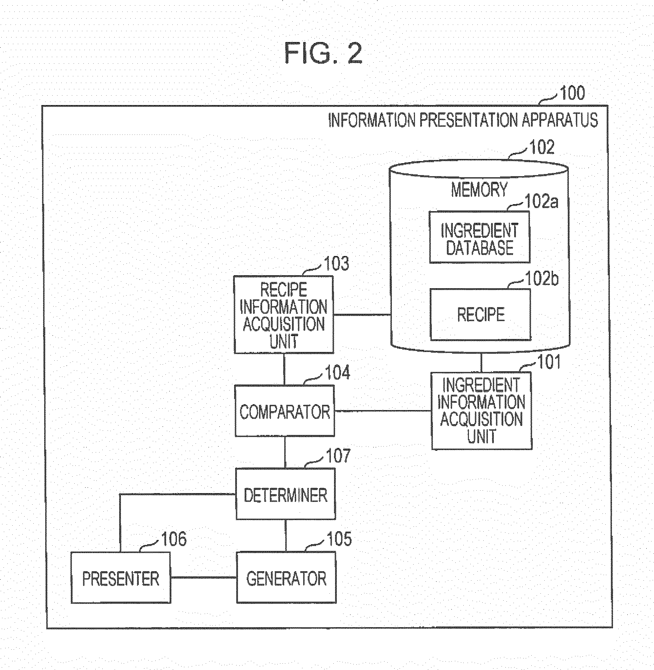

The functional configuration of the information presentation apparatus 100 is described with reference to Fig. 2.

Fig. 2 is a block diagram illustrating an example of a configuration of the information presentation apparatus 100 of the first embodiment.

Referring to Fig. 2, the information presentation apparatus 100 includes, as elements thereof, an ingredient information acquisition unit 101, a recipe information acquisition unit 103, a comparator 104, a generator 105, a presenter 106, and a determiner 107. The information presentation apparatus 100 further includes a memory 102. The information presentation apparatus 100 includes a communication circuit, although it is not illustrated in Fig. 1, and may communicate with an external server and a portable terminal through wired or wireless communications. The ingredient information acquisition unit 101, the recipe information acquisition unit 103, the comparator 104, the generator 105, the presenter 106, and the determiner 107 may be implemented with a processor or the like, which executes a program stored on the memory 102. Alternatively, the ingredient information acquisition unit 101, the recipe information acquisition unit 103, the comparator 104, the generator 105, and the determiner 107 may be implemented with a microcomputer or a dedicated circuit.

The memory 102 may include a read-only memory (ROM) that stores a program and data, and a random-access memory (RAM) that stores data when the program is executed. The memory 102 also stores an ingredient database 102a and a recipe 102b.

The ingredient database 102a is a collection of information concerning the shape, color, pattern, size, and the like of each of a variety of ingredients, and is used with the ingredient information acquisition unit 101 to acquire the type of an ingredient placed on the counter. A procedure for preparing the ingredient is written in the recipe 102b. The recipe 102b defines an amount of each ingredient in the preparation operation, the preparation method, such as how to cut the ingredient, an appropriate placement orientation of the ingredient when the ingredient is prepared (the second placement orientation). The ingredient database 102a and the recipe 102b may be pre-stored on the memory 102 in the information presentation apparatus 100 or may be obtained from a server via the Internet.

The ingredient information acquisition unit 101 acquires the type and placement orientation of an ingredient placed on a counter (first placement orientation). For example, the ingredient information acquisition unit 101 identifies the type of the ingredient by recognizing the image that the camera 200 has captured by photographing the ingredient. The ingredient information acquisition unit 101 recognizes the first placement orientation of the ingredient in the image. Based on the ingredient information acquisition unit 101, or the ingredient database 102a pre-recorded on a cloud server via the Internet (an image, for example), the ingredient information acquisition unit 101 recognizes the type and the first placement orientation of the ingredient using related art techniques, such as template matching or deep learning. For example, the ingredient information acquisition unit 101 identifies and acquires the type and the first placement orientation of the ingredient by comparing a feature point of the ingredient on the ingredient database 102a with a feature point of the ingredient in the image captured through photographing.

The recipe information acquisition unit 103 acquires a preparation method of the type of an ingredient defined by the recipe 102b and acquired by the ingredient information acquisition unit 101 and a second placement orientation (an optimum placement orientation in the preparation operation) when the acquired type of the ingredient is prepared in accordance with the preparation method.

The comparator 104 compares the second placement orientation acquired by the recipe information acquisition unit 103 with the first placement orientation acquired by the ingredient information acquisition unit 101.

The determiner 107 includes a placement orientation determiner. The placement orientation determiner determines whether the first placement orientation and the second placement orientation, compared by the comparator 104, match each other.

Based on the comparison results of the comparator 104 that has compared the first placement orientation with the second placement orientation, the generator 105 generates the rotation guidance information that prompts the preparator, who is to prepare the ingredient placed on the counter, to rotate the ingredient from the first placement orientation to the second placement orientation. The generator 105 generates preparation guidance information indicating the preparation method.

The presenter 106 presents to the preparator the rotation guidance information and the preparation guidance information (content) generated by the generator 105. For example, the presenter 106 causes a visual presenting module, such as the projector 300 (a liquid-crystal projector) or a laser light source, to output an image as these pieces of information. The preparation guidance information may be displayed in an overlay fashion on the ingredient. Alternatively, the preparation guidance information may be displayed on a liquid-crystal monitor mounted at a location that is viewable by the preparator who is preparing the ingredient. Alternatively, the preparation guidance information may be announced by a presenter, such as a speaker.

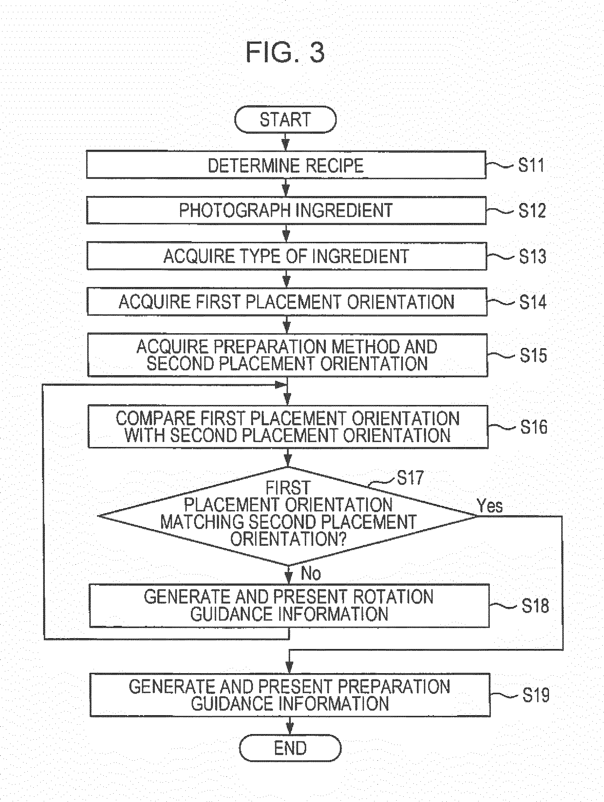

The comparator 104, the generator 105, the presenter 106, the determiner 107 and the like of the first embodiment are described in detail below with reference to Fig. 3 through Fig. 5.

### 1.2 Process of information presentation apparatus

The process of the information presentation apparatus 100 of the first embodiment is described below.

Fig. 2 is a flowchart illustrating an example of a process of the information presentation apparatus 100 of the first embodiment.

The recipe information acquisition unit 103 determines a recipe by selecting it from among recipes registered in the recipe 102b (step S11). For example, the recipe information acquisition unit 103 may determine a recipe by entering the recipe on an input unit in the information presentation apparatus 100 or a portable terminal. Alternatively, the projector 300 projects an image listing multiple recipes, and the preparator may hold his or her hand over a recipe to select the recipe in use from among the multiple recipes. The ingredient information acquisition unit 101 (the preparator information acquisition unit 108 described with reference to a second embodiment) determines which recipe the preparator holds over his or her hand from among the projected recipes in accordance with the image captured by the camera 200. The recipe information acquisition unit 103 thus determines the recipe to perform from now on from the recipes registered in the recipe 102b. In this way, the recipe information acquisition unit 103 determines the recipe in response to a gesture of the preparator. Even if the preparator's hand may be wet with water or ingredient, the recipe is decided without staining the portable terminal or the like.

The camera 200 photographs the ingredient (step S12).

The ingredient information acquisition unit 101 acquires the type of the ingredient by causing the camera 200 to photograph the ingredient (step S13). More specifically, the ingredient information acquisition unit 101 identifies and acquires the type of the ingredient by checking the shape, color, pattern, and size of the ingredient in the image captured by the camera 200 against the ingredient database 102a. The type of the ingredient is the name of a food, such as "mackerel".

The ingredient information acquisition unit 101 acquires the first placement orientation of the ingredient by photographing the ingredient with the camera 200 (step S14). The first placement orientation is a direction (angle) of a longer side of a rectangle circumscribing the outline of the ingredient with respect to a direction serving as a predetermined reference in the image captured by the camera 200 (a lateral direction of the counter, for example). For example, the first placement orientation may be 90°, for example.

The recipe information acquisition unit 103 acquires the preparation method of the type of the ingredient defined in the decided recipe and acquired by the ingredient information acquisition unit 101, and the second placement orientation when the acquired type of the ingredient is prepared in accordance with the preparation method (step S15). If the type of the ingredient acquired by the ingredient information acquisition unit 101 is "mackerel", the recipe information acquisition unit 103 acquires the preparation method of mackerel, such as filleting mackerel into three pieces, and acquires the second placement orientation in which mackerel is headed, or filleted into three pieces. The second placement orientation is a direction of the ingredient which is more easily prepared by the preparator. For example, the second placement orientation is a direction (angle) of a longer side of the rectangle circumscribing the outline of the ingredient with respect to a direction vertical to the forward direction of the preparator (for example, a lateral direction of the counter). When a mackerel is headed, the second placement orientation is a direction of the longer side (a direction extending from the head to the tail) that is aligned with the lateral direction of the counter, and is 0°, for example.

The comparator 104 compares the second placement orientation acquired by the recipe information acquisition unit 103 with the first placement orientation acquired by the ingredient information acquisition unit 101 (step S16). For example, the comparator 104 compares a second placement orientation of 0° with a first placement orientation of 20°.

The determiner 107 (specifically, the placement orientation determiner) determines whether the first placement orientation and the second placement orientation, compared by the comparator 104, match each other (step S17). More specifically, the placement orientation determiner determines whether the angles described above match each other.

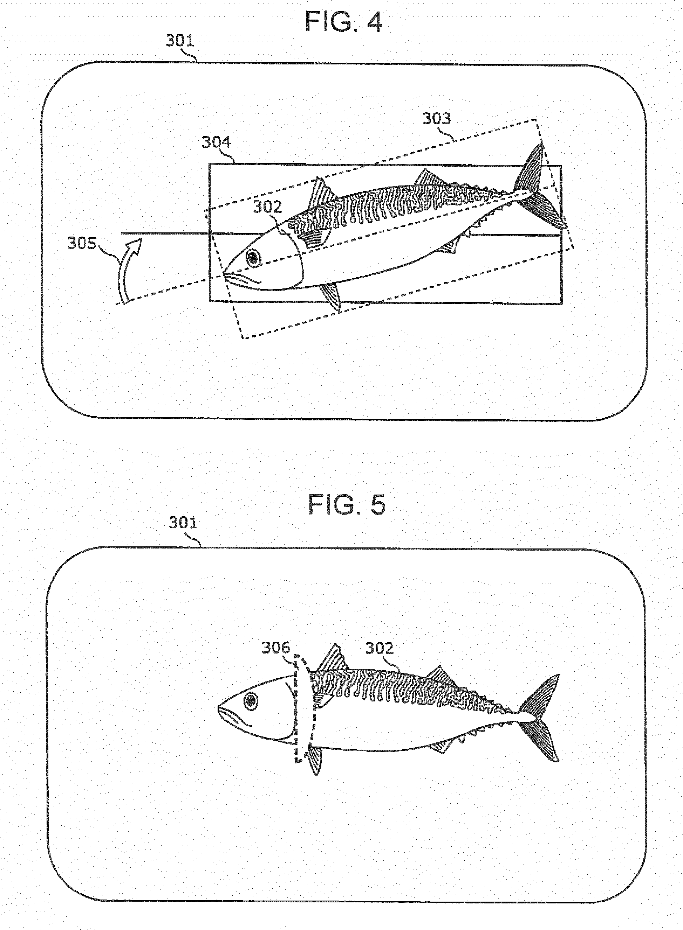

If the placement orientation determiner determines that the first placement orientation acquired by the ingredient information acquisition unit 101 fails to match the second placement orientation acquired by the recipe information acquisition unit 103 (no branch from step S107), the generator 105 generates the rotation guidance information and the presenter 106 presents the rotation guidance information (step S18). The operation in step S18 is described in detail with reference to Fig. 4.

Fig. 4 illustrates an example of the rotation guidance information. The rotation guidance information prompts the preparator, who is to prepare the ingredient placed on the counter, to rotate the ingredient if the first placement orientation of the ingredient fails to match the second placement orientation. For example, the rotation guidance information is an image that is displayed in an overlay fashion on the counter (the cutting board 301 herein) and the ingredient 302 (mackerel herein). For example, the rotation guidance information includes an image 303 indicating the first placement orientation, an image 304 indicating the second placement orientation, and an image 305 indicating a direction of rotation. The image 303 indicating the first placement orientation indicates a straight line running through the center points of shorter sides of the rectangle circumscribing the ingredient 302 (referred to as a first straight line). The image 304 indicating the second placement orientation indicates a straight line running through the center points of shorter sides of a rectangle that is aligned with the second placement orientation (referred to as a second straight line). The image 305 indicating the direction of rotation indicates an arrow mark that prompts the preparator to rotate the first straight line to the second straight line (to cause the ingredient to rotate from the first placement orientation to the second placement orientation).

Fig. 4 specifically illustrates a procedure in which the ingredient 302 placed on the cutting board 301 is filleted into three pieces. When the mackerel is first headed in the procedure, the first placement orientation of the ingredient 302 currently fails to match the second placement orientation. The generator 105 generates the rotation guidance information that helps the preparator correct the first placement orientation of the ingredient 302, and the presenter 106 causes the projector 300 to display the rotation guidance information on or near the ingredient 302. The rotation guidance information includes at least the image 305 indicating the direction of rotation, and may not necessarily include the image 303 indicating the first placement orientation and the image 304 indicating the second placement orientation. In such a case, information indicating what degrees the ingredient 302 is to be rotated may be displayed in addition to the image 305 indicating the direction of rotation.

The operations in steps S16 through S18 are iterated until the first placement orientation matches the second placement orientation. For example, if the first placement orientation of the current ingredient 302 changes, the changed first placement orientation is compared with the second placement orientation. If the changed first placement orientation fails to match the second placement orientation, the rotation guidance information prompting the preparator to rotate the changed first placement orientation to the second placement orientation is presented.

If the placement orientation determiner determines that the first placement orientation matches the second placement orientation (yes branch from step S17), the generator 105 generates the preparation guidance information and the presenter 106 presents the preparation guidance information (step S19). Since the first placement orientation matches the second placement orientation, the generator 105 generates no rotation guidance information, and the presenter 106 presents no preparation guidance information. The operation in step S19 is described in detail with reference to Fig. 5.

Fig. 5 illustrates an example of a state in which the preparation guidance information is displayed in an overlay fashion on the ingredient 302 which is aligned with the second placement orientation. The preparation guidance information is information indicating the preparation method, and, for example, is an image indicating how to cut the ingredient 302, such as cutting in round slices, cutting into chunks, or filleting in three pieces, and cutting locations in the ingredient 302. Fig. 5 illustrates an image 306 indicating a cutting location where the ingredient 302 is headed. In this way, the preparation guidance information is displayed in an overlay fashion on the ingredient 302 which is aligned with the second placement orientation that is appropriate for the preparator to prepare the ingredient 302.

As described above, the rotation guidance information is presented to prompt the preparator to rotate the ingredient placed on the counter from the first placement orientation to the second placement orientation defined in the recipe. The preparator may thus be able to rotate the ingredient to the second placement orientation in accordance with the rotation guidance information. As illustrated in Fig. 5, the ingredient 302 placed on the cutting board 301 is thus aligned with the second placement orientation, and the preparation guidance information is presented (displayed in an overlay fashion) on the ingredient 302 in the second placement orientation. The information presentation apparatus 100 thus assists the preparator in the preparation operation. The information presentation apparatus 100 may possibly control an erroneous preparation operation of the preparator and help protect the preparator from getting hurt.

### Second Embodiment

In accordance with the first embodiment, the recipe 102b defines an amount of ingredient to be used in the preparation, the preparation method, such as how to cut the ingredient, an appropriate placement orientation of the ingredient (the second placement orientation) when the ingredient is prepared. In accordance with a second embodiment, the recipe 102b further defines a positional relationship between a standing location of the preparator who is to prepare the ingredient at an appropriate placement location.

### 2.1 Configuration of information presentation apparatus

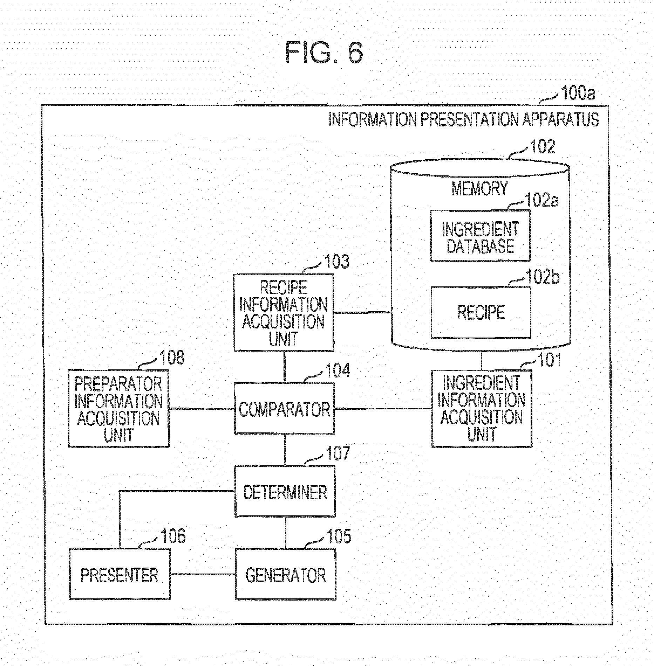

Fig. 6 is a block diagram illustrating an example of a configuration of an information presentation apparatus 100a of the second embodiment. Elements identical to those illustrated in Fig. 2 are designated with the same reference numerals, and the detailed discussion thereof is omitted herein. Referring to Fig. 6, the information presentation apparatus 100a further includes a preparator information acquisition unit 108. The following discussion focuses on a difference between the first embodiment and the second embodiment with the discussion of the same point therebetween omitted herein.

The preparator information acquisition unit 108 acquires the standing location of the preparator. The preparator information acquisition unit 108 recognizes and acquires the standing location of the preparator in the image obtained when the camera 200 photographs the ingredient. For example, the preparator information acquisition unit 108 acquires the standing location of the preparator, based on the hands, the whole body, or cookware used by the preparator, photographed in the image.

The ingredient information acquisition unit 101 acquires a placement location where an ingredient is placed on the counter (a first placement location). For example, the ingredient information acquisition unit 101 recognizes and acquires the first placement location of the ingredient in the image that is obtained when the camera 200 photographs the ingredient.

The recipe information acquisition unit 103 acquires the positional relationship between the standing location and the placement location of the type of the ingredient when the acquired type of the ingredient is prepared in accordance with the preparation method acquired and defined by the recipe 102b.

Based on the positional relationship acquired by the recipe information acquisition unit 103, the comparator 104 calculates the second placement location with respect to the standing location of the preparator acquired by the preparator information acquisition unit 108 (a placement location optimum for the preparation operation), and compares the second placement location with the first placement location acquired by the ingredient information acquisition unit 101.

The determiner 107 includes the placement orientation determiner and placement location determiner. The placement orientation determiner has been described with reference to the first embodiment, and the discussion thereof is omitted herein. The placement location determiner determines whether the first placement location and the second placement location, compared by the comparator 104, match each other.

The generator 105 further generates movement guidance information that prompts the preparator to move the ingredient from the first placement location acquired by the ingredient information acquisition unit 101 to the second placement location calculated by the comparator 104.

The presenter 106 presents to the preparator the movement guidance information generated by the generator 105.

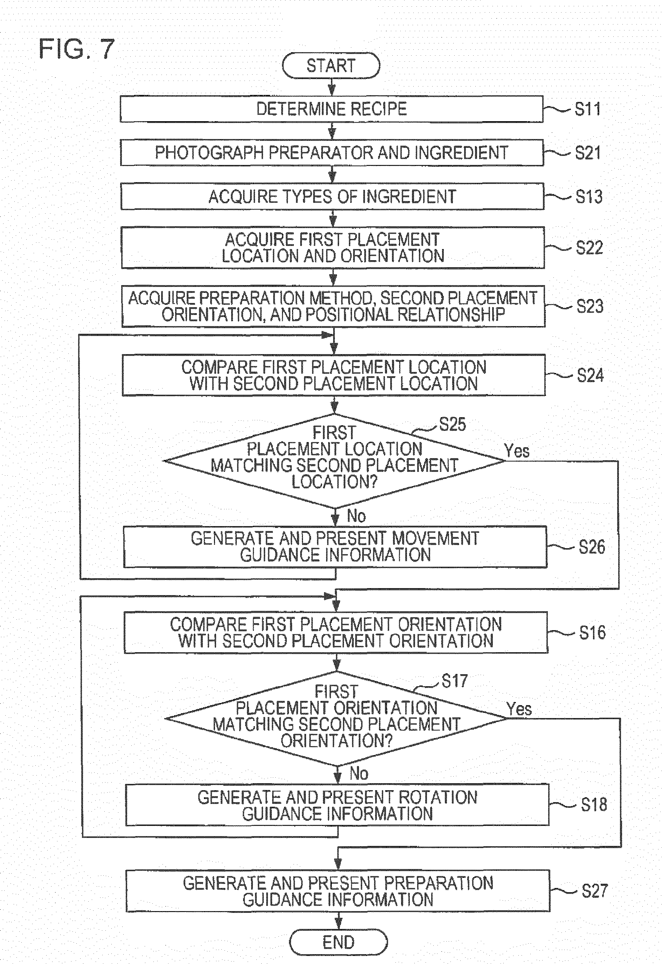

The comparator 104, the generator 105, the presenter 106 and the determiner 107 in the second embodiment are described in detail with reference to Fig. 7 through Fig. 9.

### 2.2 Process of information presentation apparatus

The process performed by the information presentation apparatus 100a of the second embodiment is described below.

Fig. 7 is a flowchart illustrating the process performed by the information presentation apparatus 100a of the second embodiment. Operations in steps S11, S13, and S16 through S18 are identical to those of the first embodiment and the discussion thereof is omitted herein.

Subsequent to the operation in step S11, the camera 200 photographs the ingredient placed on the counter and the preparator (step S21).

Subsequent to the operation in step S13, the ingredient information acquisition unit 101 acquires the first placement orientation and the first placement location when the camera 200 photographs the ingredient (step S22). The first placement location is the location of the preparator in the image that is captured when the camera 200 photographs the ingredient.

The recipe information acquisition unit 103 acquires the preparation method of the type of the ingredient defined by the selected recipe and acquired by the ingredient information acquisition unit 101, the second placement orientation at which the acquired type of the ingredient is prepared in accordance with the preparation method, the positional relationship between the standing location of the preparator, and the placement location of the type of the ingredient (step S23). The positional relationship is a relationship between the standing location of the preparator at which the preparator may more easily prepare the ingredient and the placement location of the ingredient. For example, when mackerel is headed, the positional relationship means that the gills of the mackerel are located in front of the right hand of the preparator.

Based on the positional relationship acquired by the recipe information acquisition unit 103, the comparator 104 calculates the second placement location with respect to the standing location of the preparator acquired by the preparator information acquisition unit 108, and compares the second placement location with the first placement location acquired by the ingredient information acquisition unit 101 (step S24). More specifically, the comparator 104 calculates the second placement location such that the positional relationship between the standing location of the actual preparator and the second placement location is identical to the positional relationship in the recipe between the standing location of the preparator and the placement location of the ingredient, and compares the first placement location and the second placement location.

The determiner 107, specifically the placement location determiner, determines whether the first placement location and the second placement location, compared by the comparator 104, match each other (step S25).

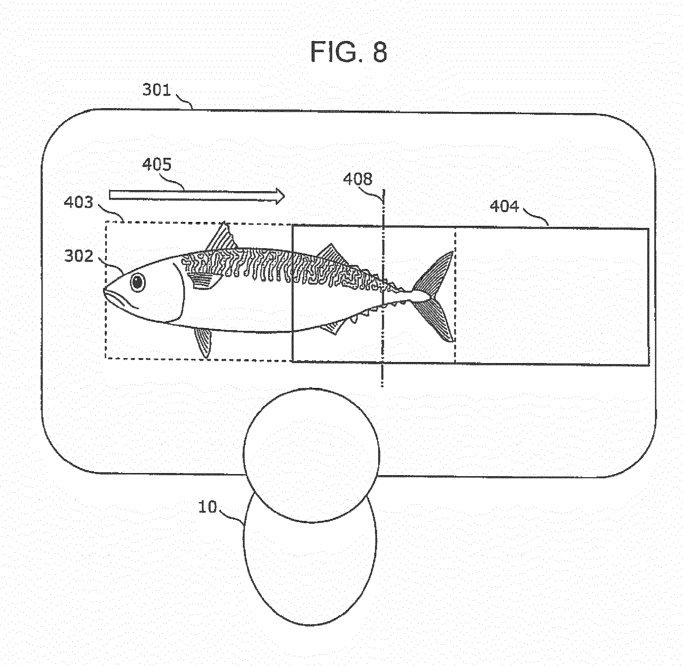

If the placement location determiner determines that the first placement location and the second placement location do not match each other (no branch from step S25), the generator 105 generates the movement guidance information and the presenter 106 presents the movement guidance information (step S26). The operation in step S26 is described in detail with reference to Fig. 8.

Fig. 8 illustrates an example of the movement guidance information. Fig. 8 schematically illustrates a preparator 10. The movement guidance information prompts the preparator 10, who is to prepare the ingredient placed on the counter, to move the ingredient from the first placement location to the second placement location if the first placement location of the ingredient placed on the counter does not match the second placement location. For example, the movement guidance information includes an image 403 indicating the first placement location, an image 404 indicating the second placement location, an image 405 indicating a movement direction, and an image 408 indicating the location where the preparation guidance information is displayed. The image 403 indicating the first placement location indicates a rectangle circumscribing the ingredient 302. The image 404 indicating the second placement location indicates a rectangle circumscribing the second placement location. The image 405 indicating the movement direction indicates an arrow mark that prompts the preparator 10 to move the ingredient 302 from the first placement location to the second placement location. The image 408 indicating the location where the preparation guidance information is displayed indicates a straight line indicating the location where the preparation guidance information is displayed.

Fig. 8 specifically illustrates a state in which the first placement location of the current ingredient fails to match the second placement location when the ingredient 302 is headed in a first step before the ingredient 302 placed on the cutting board 301 is filleted into three pieces. The generator 105 generates the movement guidance information such that the preparator 10 corrects the first placement location and the presenter 106 causes the projector 300 to display the movement guidance information in an area surrounding the ingredient 302. The movement guidance information may include at least the image 405 indicating the movement direction, and may not necessarily include the image 403 indicating the first placement location, the image 404 indicating the second placement location, and the image 408 indicating the location where the preparation guidance information is displayed. In such a case, information indicating how far the ingredient 302 is to be moved may be presented in addition to the image 405 indicating the movement direction.

Operations in steps S24 through S26 are iterated until the first placement location matches the second placement location. For example, if the first placement location of the current ingredient 302 changes, the changed first placement location is compared with the second placement location. If the changed first placement location fails to match the second placement location, the movement guidance information is presented so that the changed first placement location matches the second placement location.

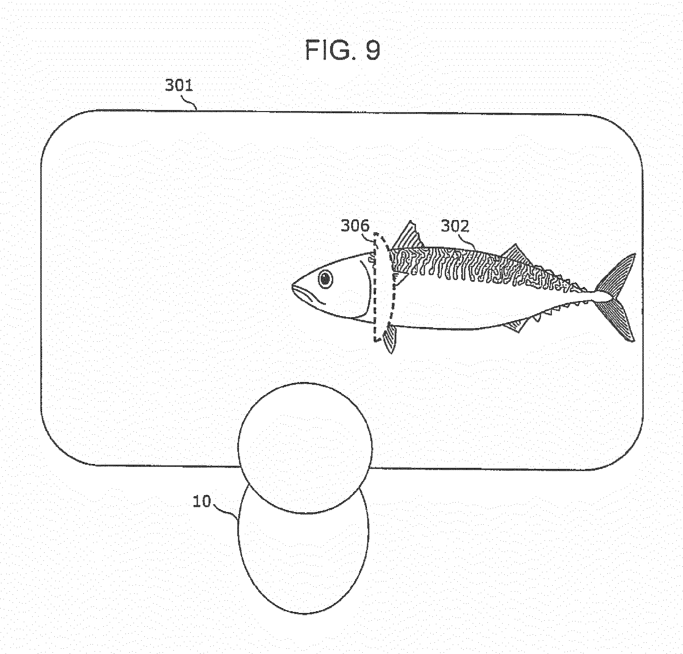

If the placement location determiner determines whether the first placement location matches the second placement location (yes branch from step S25), operations in steps S16 through S18 are performed. Since the first placement location matches the second placement location, the generator 105 generates no movement guidance information and the presenter 106 presents no movement guidance information. Further, if the placement orientation determiner determines that the first placement orientation of the ingredient matches the second placement orientation (yes branch from S17), the generator 105 generates the preparation guidance information and the presenter 106 presents the preparation guidance information (step S27). The operation in step S27 is described in detail with reference to Fig. 9.

Fig. 9 illustrates an example of a state in which the preparation guidance information is displayed in an overlay fashion on the ingredient 302 which has been shifted to the second placement location to be aligned with the second placement orientation. The preparation guidance information indicates the preparation method, and for example, is an image that indicates how to cut the ingredient in round slices, cut the ingredient in chunks, filleting the ingredient in three pieces, or a cutting location. Fig. 9 includes an image 306 indicating a cutting location where the ingredient 302 is headed. In this way, the preparation guidance information is displayed in an overlay fashion on the ingredient 302 that is placed at the second placement location appropriate for the preparator to prepare the ingredient 302 and is aligned with the second placement orientation appropriate for the preparator to prepare the ingredient 302.

As described above, the movement guidance information is presented to prompt the preparator 10 to move the ingredient placed on the counter at the first placement location to the second placement location corresponding to the positional relationship between the standing location of the preparator in the preparation method defined in the recipe and the placement location of the ingredient. The preparator 10 may thus move the ingredient to the second placement location in accordance with the movement guidance information. Since the preparation guidance information is displayed in an overlay fashion on the ingredient 302 placed at the second placement location as illustrated in Fig. 9, the preparator 10 may more easily prepare the ingredient.

### Third Embodiment

In accordance with the second embodiment, the recipe 102b defines an amount of ingredient to be used in the preparation, the preparation method, such as how to cut the ingredient, an appropriate placement orientation of the ingredient when the ingredient is prepared, and the positional relationship between the standing location of the preparator when the preparator is preparing the ingredient and the appropriate placement location of the ingredient. Further in accordance with a third embodiment, a holding location of the ingredient in the preparation is defined.

### 3.1 Configuration of information presentation apparatus

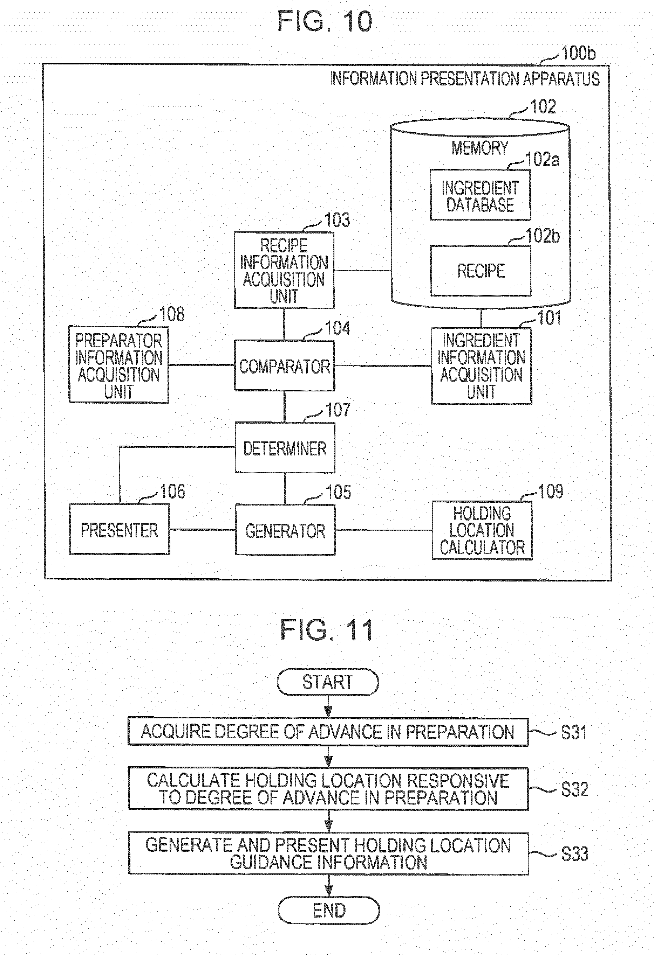

Fig. 10 is a block diagram illustrating an example of a configuration of an information presentation apparatus 100b of the third embodiment. Elements identical to those illustrated in Fig. 6 are designated with the same reference numerals, and the detailed discussion thereof is omitted herein. Referring to Fig. 10, the information presentation apparatus 100b further includes a holding location calculator 109. The following discussion focuses on a difference between the second embodiment and the third embodiment with the discussion of the same point therebetween omitted herein.

The ingredient information acquisition unit 101 acquires the degree of advance of the preparation of the ingredient.

The holding location calculator 109 calculates the holding location responsive to the degree of advance of the preparation when the ingredient is prepared in accordance with the preparation method acquired by the recipe information acquisition unit 103.

The generator 105 generates holding location guidance information that prompts the preparator to hold the ingredient at the holding location calculated by the holding location calculator 109.

The presenter 106 further presents the holding location guidance information generated by the generator 105.

The ingredient information acquisition unit 101, the generator 105, the presenter 106, and the holding location calculator 109 of the third embodiment are described in detail with reference to Fig. 11 through Fig. 13.

### 3.2 Process of information presentation apparatus

The process of an information presentation apparatus 100b of the third embodiment is described below.

Fig. 11 is a flowchart illustrating an example of the process performed by the information presentation apparatus 100b of the third embodiment. The process of Fig. 11 is performed subsequent to the operation in step S27.

The ingredient information acquisition unit 101 acquires the degree of advance of the preparation of the ingredient (step S31). The ingredient information acquisition unit 101 recognizes and acquires the degree of advance of the preparation of the ingredient (for example, an amount of cutlet) in an image captured when the camera 200 photographs the ingredient.

The holding location calculator 109 calculates the holding location of the ingredient in response to the degree of advance of the preparation when the ingredient is prepared in the preparation method acquired by the recipe information acquisition unit 103 (step S32). For example, if the preparation method is a method of cutting a carrot, the cutting location is calculated in response to the size of the carrot whose size becomes smaller as it is cut in round slices for one end thereof. The recipe 102b defines a length from one end of the ingredient to the cutting location with the ingredient held. The holding location calculator 109 calculates the cutting location by referencing the length.

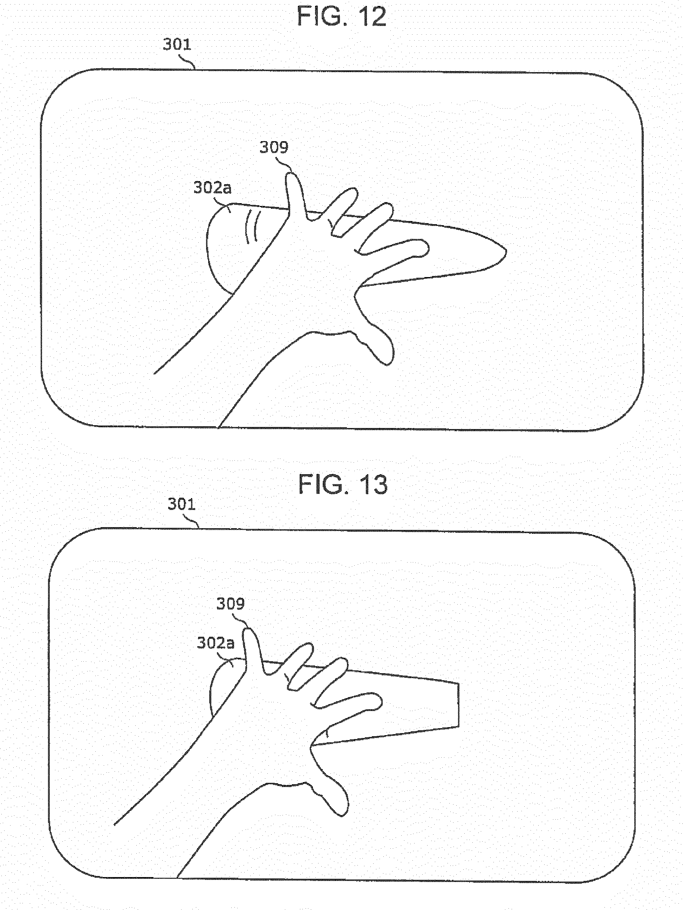

The generator 105 generates the holding location guidance information that prompts the preparator to hold the ingredient at the holding location calculated by the holding location calculator 109. The presenter 106 presents the holding location guidance information (step S33). The operation in step S33 is described in detail with reference to Fig. 12.

Fig. 12 illustrates an example of the holding location guidance information that is displayed in an overlay fashion on the holding location of the ingredient responsive to the degree of advance in the preparation of an ingredient 302a. The holding location guidance information includes an image 309 indicating the holding location, such as a computer graphic image indicating a hand. More specifically, Fig. 12 illustrates a state in which the image 309 indicating the holding location is displayed in an overlay fashion on the ingredient 302a when the ingredient 302a (such as a carrot) placed on the cutting board 301 is cut in round slices. In this way, the preparator simply recognizes the location where the image 309 indicating the holding location is displayed in an overlay fashion.

After one end of the ingredient 302a is cut in round slice, operations in step S31 through S33 are iterated. More specifically, an appropriate holding location is calculated when the ingredient 302a is cut in round slices again after the one end is cut away, and the holding location guidance information that prompts the preparator to hold the ingredient 302a at the holding location is generated and presented.

Fig. 13 illustrates how a location where the holding location guidance information is displayed in an overlay fashion changes in response to the degree of advance in the preparation. Referring to Fig. 13, when the right end portion of the ingredient 302a is cut away, the location where the image 309 indicating the holding location is displayed in an overlay fashion is shifted more leftward than the location illustrated in Fig. 12. In this way, the location where the holding location guidance information is displayed in an overlay fashion is shifted in response to the degree of advance of the preparation.

The holding location guidance information is displayed in an overlay fashion is shifted as described above in response to the degree of advance of the preparation in the preparation method defined in the recipe. This makes the ingredient to be more easily prepared, and help protect the preparator from getting hurt.

### Other Embodiments

The information presentation apparatuses of the embodiments of the disclosure have been described. The disclosure is not limited to the embodiments. It will be apparent to those skilled in the art that various changes and modifications may be made or elements in the embodiments may be combined herein without departing from the scope of the disclosure.

For example, the comparator 104 may calculate a presentation location where the rotation guidance information is presented, based on the first placement orientation and the first placement location acquired by the ingredient information acquisition unit 101, and the standing location of the preparator acquired by the preparator information acquisition unit 108, and the presenter 106 may present the rotation guidance information at the presentation location. In this way, the rotation guidance information is presented at the appropriate location in view of the first placement orientation and the first placement location of the ingredient and the standing location of the preparator, and the preparator may more easily recognize the rotation guidance information. The comparator 104 may calculate a presentation location where the movement guidance information is presented, based on the first placement orientation and the first placement location, acquired by the ingredient information acquisition unit 101, and the standing location of the preparator acquired by the preparator information acquisition unit 108, and the presenter 106 may present the movement guidance information. In this way, the movement guidance information may be presented at an appropriate location, and the preparator may more easily recognize the movement guidance information.

The ingredient information acquisition unit 101 and the preparator information acquisition unit 108 may acquire information from multiple images captured by multiple cameras 200.

In accordance with the embodiments, the ingredient information acquisition unit 101, the presenter 106, and the preparator information acquisition unit 108 are elements having the functionalities thereof in the information presentation apparatuses. The disclosure is not limited to this configuration. The ingredient information acquisition unit 101 and the preparator information acquisition unit 108 may be included in the camera 200 and the presenter 106 may be included in the projector 300.

In the embodiments, each of the information presentation apparatuses includes the determiner 107. Alternatively, each of the information presentation apparatuses may not necessarily include the determiner 107.

In the embodiments, the rotation guidance information that prompts the preparator to rotate the ingredient about an axis of rotation vertical to the plane of the counter is present. The disclosure is not limited to this arrangement. For example, the rotation guidance information that prompts the preparator to rotate the ingredient about an axis of rotation about a line extending in parallel with the counter may be presented. If the type of ingredient acquired by the ingredient information acquisition unit 101 is an onion, the recipe information acquisition unit 103 acquires a method of thinly slicing as the preparation method of onion, and acquires the second placement orientation of the onion when the onion is to be thinly sliced. In this case, the second placement orientation means an orientation of the onion that remains stable in position with a larger surface out of the relatively flat surfaces thereof being in contact with the counter. If the determiner 107 determines that the first placement orientation of the onion acquired by the ingredient information acquisition unit 101 does not match the second placement orientation acquired by the recipe information acquisition unit 103, the generator 105 generates the rotation guidance information that prompts the preparator to rotate the onion from the first placement orientation to the second placement orientation in which the onion is on the countered with the largest relatively flat surface thereof being in contact with the counter, and the presenter 106 presents the rotation guidance information. In this way, the rotation guidance information that prompts the preparator to rotate the ingredient about an axis of rotation that is in parallel with the counter.

The second placement orientation may be an orientation of the onion in which the center of gravity of the onion is on the counter side. In such a case, the ingredient information acquisition unit 101 acquires the position of the center of gravity of the current ingredient in the first placement orientation from the image captured when the camera 200 photographs the ingredient. The rotation guidance information prompting the preparator to rotate the ingredient such that the center of gravity of the current ingredient is on the counter side is generated, and presented.

The determiner 107 in the information presentation apparatus 100a of Fig. 6 has the functionality to determine whether the first placement orientation and the second placement orientation, compared by the comparator 104, match each other, and the functionality to determine whether the first placement location and the second placement location, compared by the comparator 104, match each other. The disclosure is not limited to this configuration. The determiner 107 in each information presentation apparatus may include a placement location determiner that determines whether the placement location and the second placement location, compared by the comparator 104, match each other. The process of the information presentation apparatus in this configuration is identical the process of the flowchart of Fig. 7 without steps S16 through S18, and S27.

In accordance with the embodiments, the first placement location and the second placement location are compared, and the movement guidance information is generated and presented before the first placement orientation and the second placement orientation are compared with each other, and the rotation guidance information is generated and presented. The disclosure is not limited to this process. Alternatively, the first placement orientation and the second placement orientation are compared with each other, and the rotation guidance information is generated and presented before the first placement location and the second placement location are compared, and the movement guidance information is generated and presented. More specifically, the placement orientation of the ingredient placed on the counter is corrected before the placement location is corrected.

The information presentation apparatus may be implemented as a server. By implementing the camera 200 and the projector 300 as a client apparatus, the server and the client apparatus may form a client server system.

The disclosure may be embodied not only as the information presentation apparatus but also as an information presentation method including operations to be performed in steps (process) performed by each element forming the information presentation apparatus.

As illustrated in Fig. 3, the information presentation method includes acquiring the type and the first placement orientation of the ingredient placed on the counter (steps S13 and S14), acquiring a preparation method, for the type of the ingredient, defined by the recipe, and the second placement orientation of the acquired type of the ingredient when the ingredient is prepared in accordance with the preparation method (step S15), comparing the acquired second placement orientation with the acquired first placement orientation (step S16), generating preparation guidance information indicating the preparation method and generating rotation guidance information that prompts the preparator to rotate the ingredient from the first placement orientation to the second placement orientation in accordance with comparison results of the first placement orientation with the second placement orientation, and presenting the generated rotation guidance information and the generated preparation guidance information (steps S18 and S19).

The steps may be performed by a computer (or compute system). The disclosure may be implemented as a program that causes a computer to perform the steps included in the method. The disclosure may also be implemented as a non-transitory computer-readable recording medium, such as a compact disk read-only memory (CD-ROM) that has recorded the program.

If the disclosure is implemented as a program (software), each step is performed by executing the program using a hardware resource, such as a central processing unit (CPU) of the computer, a memory, and an input and output circuit. More specifically, each step is performed by the CPU that retrieves data from the memory or the input and output circuit, and outputs arithmetic processing results to the memory or the input and output circuit.

Multiple elements included in each of the information presentation apparatuses of the embodiments may be implemented as a dedicated or general-purpose circuit. The elements may be implemented in a single circuit or multiple circuits.

Multiple elements included in each of the information presentation apparatuses may be implemented as large scale integration that is an integrated circuit (IC). The elements may be implemented in individual chips or some or all of the elements may be implemented in a single chip. The LSI may be referred to as an IC, a system LSI, a super LSI, or an ultra LSI, depending on a difference in the degree of integration.

The integrated circuit may be not only an LSI, but also a dedicated circuit or a general-purpose processor. A field programmable gate array (FPGA) or a reconfigurable processor may be used. The reconfigurable processor is reconfigurable in terms of the circuit cell connection and configuration of the internal LSI.

In accordance with the embodiments, the preparation method of ingredient is cutting the ingredient. The preparation method includes a variety of ways to change an ingredient in shape or state, such as beating, stretching, rubbing, smashing, peeling, turning over, or crushing.

In accordance with the embodiments, the degree of advance of the preparation is acquired from the image that is captured by the camera 200 that photographs the ingredient. The method of acquiring the degree of advance of the preparation is not limited to this. More specifically, the degree of advance of the preparation may be acquired in response to an input entered by the preparator. For example, the preparator enters, to an input unit, information that the preparation has been well in progress. The ingredient information acquisition unit 101 acquires the degree of advance by acquiring the information indicating that the preparation has been well in progress.

An embodiment obtained by introducing changes and modifications apparent to those skilled in the art in each of the above embodiments and an embodiment obtained by combining elements of the embodiments fall within the scope of the disclosure.

The disclosure is useful as an information presentation apparatus that assists a preparator in a preparation operation.

## Claims

1. An information presentation apparatus comprising:
an ingredient information acquirer that acquires a type and a first placement orientation of an ingredient placed on a counter;
a recipe information acquirer that acquires a preparation method, for the type of the ingredient, defined by a recipe, and a second placement orientation of the acquired type of the ingredient when the ingredient is prepared in accordance with the preparation method;
a comparator that compares the second placement orientation acquired by the recipe information acquirer with the first placement orientation acquired by the ingredient information acquirer;
a generator that generates preparation guidance information indicating the preparation method and generates rotation guidance information that prompts a preparator to rotate the ingredient from the first placement orientation to the second placement orientation in accordance with comparison results provided by the comparator that has compared the first placement orientation with the second placement orientation; and
a presenter that presents the rotation guidance information and the preparation guidance information, generated by the generator.

2. The information presentation apparatus according to Claim 1, further comprising a placement orientation determiner that determines whether the first placement orientation and the second placement orientation, compared by the comparator, match each other,
wherein the presenter presents the rotation guidance information if the placement orientation determiner determines that the first placement orientation and the second placement orientation do not match each other, and
wherein the presenter presents the preparation guidance information if the placement orientation determiner determines that the first placement orientation and the second placement orientation match each other.

3. The information presentation apparatus according to Claims 1 or 2, further comprising a preparator information acquirer that acquires a standing location of the preparator,
wherein the ingredient information acquirer acquires a first placement location of the ingredient,
wherein the recipe information acquirer acquires a positional relationship between the standing location of the preparator when the acquired type of the ingredient is prepared in accordance with the preparation method defined by the recipe and a placement location of the acquired type of the ingredient,
wherein based on the positional relationship acquired by the recipe information acquirer, the comparator calculates a second placement location concerning the standing location of the preparator that the preparator information acquirer has acquired, and compares the second placement location with the first placement location acquired by the ingredient information acquirer,
wherein the generator generates movement guidance information that prompts the preparator to move the ingredient from the first placement location to the second placement location, based on comparison results provided by the comparator that has compared the first placement location with the second placement location, and
wherein the presenter presents the movement guidance information generated by the generator.

4. The information presentation apparatus according to Claim 3, further comprising a placement location determiner that determines whether the first placement location and the second placement location, compared by the comparator, match each other,
wherein the presenter presents the movement guidance information if the placement location determiner determines that the first placement location and the second placement location do not match each other, and
wherein the presenter presents the preparation guidance information if the placement location determiner determines that the first placement location and the second placement location match each other.

5. The information presentation apparatus according to any one of Claims 1 to 4, further comprising a holding location calculator that calculates a holding location of the ingredient that accounts for a degree of advance of preparation of the ingredient when the ingredient is prepared in accordance with the preparation method,
wherein the ingredient information acquirer acquires the degree of advance of the preparation of the ingredient,
wherein the generator generates holding location guidance information that prompts the preparator to hold the ingredient at the holding location calculated by the holding location calculator, and
wherein the presenter presents the holding location information generated by the generator.

6. The information presentation apparatus according to any one of Claims 1 to 5, further comprising a preparator information acquirer that acquires a standing location of the preparator,
wherein the ingredient information acquirer acquires a first placement location of the ingredient,
wherein the comparator calculates a presentation location where the rotation guidance information is presented, based on the first placement orientation acquired by the ingredient information acquirer, the first placement location, and the standing location of the preparator acquired by the preparator information acquirer, and
wherein the presenter presents the rotation guidance information at the presentation location.

7. An information presentation method comprising:
acquiring a type and a first placement orientation of an ingredient placed on a counter;
acquiring a preparation method, for the type of the ingredient, defined by a recipe, and a second placement orientation of the acquired type of the ingredient when the ingredient is prepared in accordance with the preparation method;
comparing the acquired second placement orientation with the acquired first placement orientation;
generating preparation guidance information indicating the preparation method and generating rotation guidance information that prompts a preparator to rotate the ingredient from the first placement orientation to the second placement orientation in accordance with comparison results of the first placement orientation with the second placement orientation; and
presenting the generated rotation guidance information and the generated preparation guidance information.
